(19) Europäisches Patentamt
European Patent Office
Office européen des brevets

(11) **EP 3 456 254 A1**

(12) **EUROPEAN PATENT APPLICATION**

(43) Date of publication:
**20.03.2019 Bulletin 2019/12**

(21) Application number: **17191615.8**

(22) Date of filing: **18.09.2017**

(51) Int Cl.:
*A61B 5/022* [(2006.01)]       *A61B 5/00* [(2006.01)]
*A61B 5/021* [(2006.01)]       *A61B 5/08* [(2006.01)]
*A61B 5/113* [(2006.01)]

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Koninklijke Philips N.V.
5656 AE Eindhoven (NL)**

(72) Inventors:
• **GELISSEN, Jozef Hubertus
5656 AE Eindhoven (NL)**
• **DE GROOT, Koen Theo Johan
5656 AE Eindhoven (NL)**
• **RADHA, Mustafa Ghassan
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **DEVICE FOR DETERMINING DIASTOLIC BLOOD PRESSURE OF A SUBJECT**

(57)     The invention relates to a device (1) for determining diastolic blood pressure in an unobtrusive and painless way such that it can be determined, for instance, during sleep. A PPG signal of the skin of a subject is provided by, for instance, a PPG sensor (4), which has been measured at a measurement location, while a force applied to the skin at a force application location has been increased, wherein the measurement location and the force application location are the same or the force application location is proximal relative to the measurement location. A force value of the increasing force is determined by a force value determination unit (6), at which an amplitude of an oscillating component of the PPG signal starts to diminish in reaction to the increasing force, wherein this force value is used by a blood pressure determination unit (8) for determining the diastolic arterial blood pressure.

FIG. 1

EP 3 456 254 A1

**Description**

FIELD OF THE INVENTION

The invention relates to a device, method and computer program for determining diastolic blood pressure of a subject.

BACKGROUND OF THE INVENTION

Blood pressure cuffs are often used for determining the diastolic blood pressure of a subject. However, measuring the diastolic blood pressure by using a blood pressure cuff, which is firmly wrapped around an upper arm of the subject, is perceived as being painful. Moreover, it is nearly impossible to obtain indicative 24 hour blood pressure results, without disturbing the subject's sleep, because the blood pressure measurements require cuff inflations.

SUMMARY OF THE INVENTION

It is therefore regarded as being an object of the present invention to provide a device, method and computer program which allow for an improved determination of diastolic blood pressure of a subject.

In a first aspect of the present invention a device for determining diastolic blood pressure of a subject is presented, wherein the device comprises:

- a photoplethysmography (PPG) signal providing unit for providing a PPG signal of the skin of the subject, which has been measured at a measurement location, while a force applied to the skin at a force application location has been increased, wherein the measurement location and the force application location are the same or the force application location is proximal relative to the measurement location,
- an oscillating component determination unit for determining an oscillating component of the PPG signal by high-pass filtering the PPG signal,
- a force value determination unit for determining a force value of the increasing force at which the amplitude of the oscillating component of the PPG signal starts to diminish in reaction to the increasing force,
- an assignments providing unit for providing assignments between force values, at which an amplitude of an oscillating component of a PPG signal starts to diminish in reaction to the increasing force, and diastolic arterial blood pressures, and
- a blood pressure determination unit for determining the diastolic arterial blood pressure based on the determined force value and the provided assignments.

The measurement of a PPG signal is unobtrusive and not painful. Already this leads to an improved determination of the diastolic blood pressure. Moreover, only very little force needs to be applied for determining the force value of the increasing force at which the amplitude of the oscillating component of the PPG signal starts to diminish in reaction to the increasing force such that, if the force is automatically applied, the force can be applied without disturbing a night rest of the subject. This further improves the determination of the diastolic blood pressure.

The subject is a human being or an animal. Moreover, the PPG signal providing unit can be a storing unit in which the PPG signal is stored and from which the PPG signal can be retrieved for providing the same. The PPG signal providing unit can also be a receiving unit for receiving the PPG signal from a PPG sensor measuring the PPG signal and for providing the received PPG signal. The PPG signal providing unit can also be the PPG sensor itself.

The assignments providing unit can be a storing unit in which the assignments are stored and from which the assignments can be retrieved for providing the same. Moreover, the assignments providing unit can be a receiving unit for receiving the assignments from another device in which the assignments may have been stored and for providing the received assignments. The force values of these assignments correspond to force values, which have been or would be measured at a force application location being the same or proximal to a measurement location at which the PPG signal has been measured or would be measured. Preferentially, the assignments have been determined in a calibration or training procedure, wherein for one or several subjects force values of the increasing force, at which the amplitude of the oscillating component of the PPG signal starts to diminish in reaction to the increasing force, have been determined, while the corresponding diastolic arterial blood pressures were known from, for instance, standard blood pressure cuff measurements, and wherein the determined force values and the corresponding known diastolic arterial blood pressures are used for determining the assignments. A fitting procedure might be used for determining further assignments. The assignments can also be determined by using a simulation. The assignments may be provided, for instance, as a function, a table, et cetera.

In an embodiment the device further comprises a force applicator for automatically applying the increasing force to the skin while measuring the PPG signal of the skin. However, in another embodiment the pressure may be applied manually by a user, for instance, by pressing on the device, particularly on a PPG sensor, arranged on the skin. The device preferentially further comprises a force sensor for determining the increasing force applied to the skin.

In an embodiment the force value determination unit is adapted to only consider changes of the amplitude of the oscillating component, which are larger than a predefined threshold, for determining the force value at which the amplitude of the oscillating component of the PPG signal starts to diminish in reaction to the increasing force. The changes are preferentially changes with respect to consecutive amplitudes. If changes are not considered, which are not larger than the predefined threshold, it can be prevented that fluctuations in the amplitude of the oscillating component of the PPG signal, which are not related to the application of the increasing force, lead to a wrong determination of the force value of the increasing force at which the amplitude of the oscillating component of the PPG signal starts to diminish in reaction to the increasing force. This can lead to a further improved determination of the diastolic blood pressure.

In an embodiment the force value determination unit is adapted to determine the force value such that it is the force value of the increasing force at which the amplitude of the oscillating component of the PPG signal starts to monotonically diminish in reaction to the increasing force. Moreover, in an embodiment the oscillating component determination unit is adapted to high-pass filter the PPG signal such that the oscillating component is the pulsatile component of the PPG signal. The PPG signal comprises a pulsatile component, which might also be regarded as being a pulsatile physiological waveform, attributed to cardiac synchronous changes in the blood volume with each heart beat, and which is superimposed on a slowly varying baseline with various lower frequency components attributed to respiration, sympathetic nervous system activity and thermoregulation. For more details regarding the different components of the PPG signal reference is made to the article "Photoplethysmography and its application in clinical physiological measurement" by John Allen, Physiological Measurement, IOP Publishing, 28, R1-R39 (2007), which is herewith incorporated by reference. In an embodiment the oscillating component determination unit is adapted to determine the oscillating component such that it is or corresponds to the pulsatile component attributed to the cardiac synchronous changes in the blood volume. In order to achieve this, the oscillating component determination unit is preferentially adapted to allow components of the PPG signal, which have a frequency being equal to or larger than 0.3, further preferred equal to or larger than 0.4 and even further preferred equal to larger than 0.5 to pass the high-pass filtering. However, in another embodiment all smaller frequencies might pass the high-pass filtering such that at least a non-oscillating component, i.e. a DC component, is removed from the signal which is used for further processing.

In an embodiment the PPG signal providing unit is adapted to provide a further PPG signal of the skin of the subject, which has been measured at a further measurement location, while the force applied to the skin at the force application location is increased, wherein the further measurement location is proximal relative to the force application location, wherein the oscillating component determination unit is adapted to high-pass filter the further PPG signal for determining an oscillating component of the further PPG signal, wherein the force value determination unit is adapted to determine the force value of the increasing force at which the amplitude of the oscillating component of the PPG signal starts to diminish in reaction to the increasing force by determining when a relation between the amplitude of the oscillating component of the PPG signal and the amplitude of the oscillating component of the further PPG signal starts to change. Thus, the PPG signal providing unit can comprise, besides a first PPG sensor for measuring the PPG signal, a further PPG sensor for measuring a further PPG signal of the skin of the subject at a further measurement location, while the force applied to the skin at the force application location is increased, wherein the further measurement location is proximal relative to the force application location. The increase of the force preferentially starts with a zero force. Thus, when the force increase starts, both PPG signals are substantially not influenced by the force application and changes in the amplitude of the oscillating components of both PPG signals, if present at all, are generally only caused by breathing. However, it is also possible that the changes in the amplitude are caused by other effects like a body's temperature regulation, movements, especially posture changes, et cetera. These possibly present changes should be similar for both signals, i.e. the relation between the amplitudes of the oscillating components of the two PPG signals should be substantially constant. If the force is further increased, at a certain force value the amplitude of the oscillating component of the PPG signal, which has been measured at the measurement location, which coincides with the force application location or which is distal to the force application location, starts to diminish. However, the amplitude of the oscillating component of the other PPG signal is not influenced or less influenced by the application of the force, because the other PPG signal has been measured at the further measurement location being proximal relative to the force application location. This leads to a change in a relation between the amplitudes of the oscillating components of the two PPG signals which can be determined, in order to determine the force value of the increasing force at which the amplitude of the oscillating component of the PPG signal, which has been measured at the force application location or distal to the force application location, starts to diminish in reaction to the increasing force. This allows for an accurate determination of the force value and hence for an accurate determination of the diastolic arterial blood pressure, even if the PPG signal is strongly influenced by breathing.

In an embodiment the force value determination unit is adapted to correct the amplitude of the oscillating component of the PPG signal for breathing influences and to determine the force value at which the corrected amplitude of the oscillating component of the PPG signal starts to diminish in reaction to the increasing force. In particular, the device further comprises a breathing indicator providing unit for providing a breathing indicator being indicative of the breathing behavior of the subject, wherein the force value determination unit is adapted to determine the breathing influences based on the provided breathing indicator and to use these determined breathing influences for correcting the amplitude of the oscillating component of the PPG signal which has been measured while the force applied to the skin was increased. For instance, the PPG signal providing unit can be adapted to also provide a PPG signal which has been measured before the increasing force is applied to the skin, wherein the breathing indictor providing unit can be adapted to determine the breathing indicator based on the provided PPG signal. It is also possible that the breathing indicator providing unit comprises a breathing sensor for measuring a breathing indicator. A breathing indicator is, for instance, a variable over time which changes with the breathing, i.e., for instance, an amplitude changing in accordance with the breathing over time. The breathing indicator providing unit can comprise any sensor being able to provide a signal from which information about breathing can be determined. For instance, the breathing indicator providing unit can comprise a heart rate sensor, wherein the breathing indicator providing unit can be adapted to determine breathing from the inter-beat-interval provided by the heart rate sensor. The breathing indicator can also comprise an accelerometer, wherein the breathing indicator providing unit can be adapted to determine the breathing indicator based on an accelerometer signal provided by the accelerometer. The force value determination unit can be adapted to model the breathing influences based on the provided breathing indicator and to use the modeled breathing influences for correcting the amplitude of the oscillating component of the PPG signal which has been measured while the force applied to the skin was increased. By considering the breathing for determining the diastolic arterial blood pressure the determination of the diastolic arterial blood pressure can be further improved.

The PPG signal providing unit preferentially comprises a PPG sensor being adapted to use green light for measuring the PPG signal. It has been found that, if green light is used for measuring the PPG signal, the determination of the diastolic arterial blood pressure can be even further improved. Moreover, the PPG signal providing unit preferentially comprises a reflective PPG sensor or a transmissive PPG sensor for measuring the PPG signal.

In another aspect of the present invention a method for determining the diastolic arterial blood pressure of a subject is presented, wherein the method comprises:

- providing a PPG signal of the skin of the subject, which has been measured at a measurement location by using a PPG signal providing unit, while a force applied to the skin at a force application location is increased, wherein the measurement location and the force application location are the same or the force application location is proximal relative to the measurement location,
- determining an oscillating component of the PPG signal by high-pass filtering the PPG signal by using an oscillating component determination unit,
- determining a force value of the increasing force at which the amplitude of the oscillating component of the PPG signal starts to diminish in reaction to the increasing force by using a force value determination unit,
- providing assignments between force values, at which an amplitude of an oscillating component of a PPG signal starts to diminish in reaction to the increasing force, and diastolic arterial blood pressures by using an assignments providing unit, and
- determining the diastolic arterial blood pressure based on the determined force value and the provided assignments by using a blood pressure determination unit.

In a further aspect of the present invention a computer program for determining the diastolic arterial blood pressure of a subject is presented, wherein the computer program comprises program code means for causing a device for determining the diastolic arterial blood pressure of the subject as defined in claim 1 to carry out the steps of the method for determining the diastolic arterial blood pressure of the subject as defined in claim 14, when the computer program is run on a computer controlling the device.

It shall be understood that the device of claim 1, the method of claim 14 and the computer program of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily an embodiment of a device for determining diastolic blood pressure of a subject,

Fig. 2 illustrates schematically and exemplarily a preferred position of the device while measuring a PPG signal,

Fig. 3 illustrates schematically and exemplarily a start of a diminishing of an amplitude of an oscillating component of a PPG signal in reaction to an increasing force,

Fig. 4 illustrates exemplarily assignments between force values, at which an amplitude of an oscillating component of a PPG signal starts to diminish in reaction to an increasing force, and diastolic arterial blood pressures,

Fig. 5 shows a flowchart exemplarily illustrating an embodiment of a method for determining the diastolic arterial blood pressure of a subject,

Fig. 6 shows exemplarily assignments between force values, at which an amplitude of an oscillating component of a PPG signal becomes zero in reaction to an increasing force, and systolic arterial blood pressures,

Fig. 7 illustrates schematically and exemplarily a further embodiment of a device for determining the diastolic arterial blood pressure of a subject,

Fig. 8 exemplarily illustrates how a user could apply pressure to a device for determining the diastolic arterial blood pressure of a subject, and

Fig. 9 schematically and exemplarily illustrates a further embodiment of the device for determining the diastolic arterial blood pressure of a subject, which comprises two PPG sensors.

## DETAILED DESCRIPTION OF EMBODIMENTS

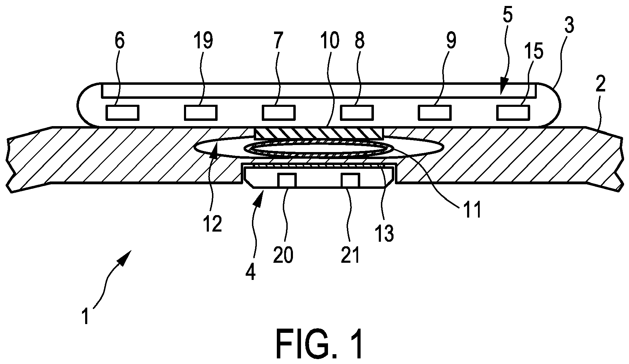

Fig. 1 shows schematically and exemplarily an embodiment of a device for determining diastolic blood pressure of a user being a person. The device 1 comprises a PPG sensor 4 for measuring a PPG signal of the skin of the user at a measurement location, while a force applied to the skin at a force application location is increased, wherein in this embodiment the measurement location and the force application location are the same. In this embodiment the PPG sensor 4 is adapted to use green light for generating the PPG signal. The PPG sensor 4 can be regarded as being a blood pulse volumetric sensor, wherein the underlying concept is pulse oximetry which tracks the average skin tone which varies with the blood volume and blood oxygenation. The PPG sensor 4 comprises a light emitting diode (LED) 20 which shines light on the skin. The PPG sensor 4 further comprises a photodiode 21 for measuring the amount of light reflected by the skin, wherein during each cardiac cycle the amount of reflected light varies as more or less blood flows through the arterioles. The variation in skin tone over time contains information on the blood volume and the photodiode 21 converts the reflected light into an electrical signal, i.e. into the PPG signal.

The device 1 is a wrist worn device including a flexible strap or wrist band 2 having two ends, wherein securement means are present for circumferentially fasten the strap around the wrist. In this embodiment the PPG sensor 4 is integrated in the wrist band 2. The device 1 further comprises a force applicator for applying the increasing force to the skin while measuring the PPG signal of the skin. In this embodiment the force applicator, which might also be regarded as being a compression means, comprises a distensible airbag 11 in a cavity 12 and an air pump and valve combination 10 for inflating and deflating the airbag 11.

The PPG sensor 4 is arranged within the device 1 such that it is in direct contact with the skin of the wrist of the user, when the device 1 is worn by the user. Moreover, the force applicator is arranged within the device 1 such that it applies the force onto the PPG sensor 4 and thereby on the measurement location on the skin where the PPG signal is measured. The force applicator hence pushes the underlying PPG sensor onto the skin by exerting a force on the underlying PPG sensor 4. In other embodiments other means for applying the force can be used. For instance, a force applicator can be used, which is adapted to shrink the inner circumference of the wrist band 2, in order to apply an increasing force to the skin of the user's wrist.

The device 1 further comprises a force sensor 13 for measuring the increasing force applied to the skin. The force sensor 13 is arranged in between the force applicator 10, 11 and the PPG sensor 4. The force sensor 13 can include, for instance, a force transducer, a strain gauge or a piezoelectric sensor. However, the force sensor 13 can also comprise other means for measuring the increasing force applied to the skin.

The device 1 further comprises an oscillating component determination unit 19 for determining an oscillating component of the PPG signal by high-pass filtering the PPG signal. In this embodiment the oscillating component determination unit 19 is adapted to use a cut-off frequency of 0.5 Hz for the high-pass filtering. In particular, the oscillating component determination unit 19 can be adapted to high-pass filter the PPG signal such that the oscillating component is the pulsatile component of the PPG signal.

The device 1 further comprises a force value determination unit 6 for determining a force value of the increasing force at which the amplitude of the oscillating component of the PPG signal starts to diminish in reaction to the increasing force. For instance, the force value determination unit 6 can be adapted to only consider changes of the amplitude of the oscillating component, which are larger than a predefined threshold, for determining the force value at which the amplitude of the oscillating component of the PPG signal starts to diminish in reaction to the increasing force. The changes are preferentially changes with respect to consecutive amplitudes of the oscillating component. If changes are not considered, which are not larger than the predefined threshold, it can be prevented that fluctuations in the amplitude of the oscillating component of the PPG signal, which are not related to the application of the increasing force, lead to a wrong determination of the force value of the increasing force at which the amplitude of the oscillating component of the PPG signal starts to diminish in reaction to the increasing force. The predefined threshold can be determined by, for instance, calibration measurements during which the increasing force is not applied and changes of the amplitude of the oscillating component are determined, wherein these changes are than not caused by the increasing force and can define the threshold.

Moreover, the force value determination unit 19 can be adapted to determine the force value such that it is the force value of the increasing force at which the amplitude of the oscillating component of the PPG signal starts to monotonically diminish in reaction to the increasing force.

Furthermore, before determining the force value, the amplitude of the oscillating component of the PPG signal can be corrected for breathing influences. For this reason the device 1 comprises a breathing indicator providing unit 15 for providing a breathing indicator being indicative of the breathing behavior of the subject, wherein the force value determination unit 6 is adapted to determine the breathing influences based on the provided breathing indicator and to correct the oscillating component of the PPG signal for these determined breathing influences. For instance, the force value determination unit 6 can be adapted to model the breathing influences based on the provided breathing indicator and to use the modeled breathing influences for correcting the amplitude of the oscillating component of the PPG signal which has been measured while the force applied to the skin was increased. The breathing indicator is preferentially a value or a signal which changes over time in accordance with the user's breathing. The breathing indicator providing unit 15 can be, for instance, an accelerometer which provides an accelerometer signal in accordance with the breathing. The breathing indicator is preferentially measured at or preferentially corresponds to a time before the increasing force is applied to the skin, in order to obtain a breathing indicator which is less likely corrupted by the increasing applied force. The breathing indicator providing unit 15 can also comprise another sensor like a sensor for measuring the heart rate and/or the inter-beat-interval, wherein the breathing indicator providing unit 15 can be adapted to derive the breathing indicator from this kind of sensor. For deriving the breathing indicator from a measurement of the heart rate and/or the inter-beat-interval known techniques can be used like the techniques disclosed in the article "Developing an algorithm for pulse oximetry derived respiratory rate (RRoxi): a healthy volunteer study", Paul S. Addison et al., volume 26, pages 45 to 51, Journal of Clinical Monitoring and Computing (2012), which is herewith incorporated by reference.

The breathing indicator providing unit 15 can also be a receiving unit for receiving the breathing indicator from another means measuring breathing information like a breathing belt. In fact, the breathing indicator providing unit 15 can comprise or receive breathing information from any means which allows measuring the breathing behavior of the subject, especially the breathing rate.

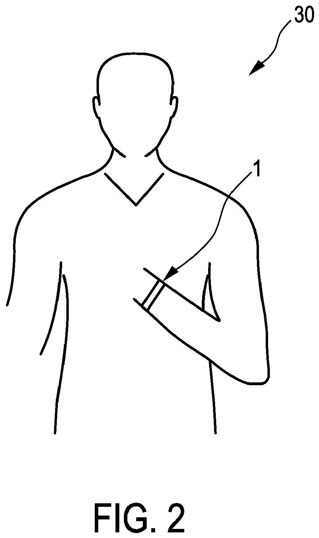

Besides breathing, the oscillating component of the PPG signal can be influenced by other factors like posture (orthostatic pressure), arm position, obstruction of the arterial path if, for instance, the user lies on his/her arm, and vasomotion. In order to control the posture, i.e. the orthostatic pressure, the arm position and a possible obstruction of the arterial path, the measurement of the PPG signal and also of the breathing indicator is preferentially standardized. Preferentially, the measurement of the PPG signal and also of the breathing indicator is carried out, while the user 30 is sitting or standing and the wrist is placed at heart height as schematically and exemplarily illustrated in Fig. 2.

The breathing indicator providing unit 15 can also comprise a further PPG sensor for measuring a further PPG signal, wherein this further PPG signal can be used for determining the breathing behavior, i.e. for determining the breathing indicator being indicative of the breathing behavior. This further PPG signal used for determining the breathing behavior can be measured before applying the force to the skin or, if the force application does not influence the further PPG signal, while the force is applied to the skin. It is also possible that the PPG sensor 4 measures a PPG signal before the increasing force is applied to the skin, wherein the force value determination unit 6 can be adapted to determine the breathing influences based on this PPG signal and to use these determined breathing influences for correcting the amplitude of the oscillating component of the PPG signal which is then measured while the force applied to the skin is increased. In this case any further sensor, i.e. a breathing sensor, for measuring a signal from which the breathing behavior can be derived is preferentially not present. The breathing indicator providing unit 15 can in this case derive and provide the breathing indicator being indicative of the breathing behavior based on the PPG signal measured by the PPG sensor 4 before applying the increasing force. In particular, since the breathing rate is normally about 12 breaths per minute, before applying the increasing force to the skin and measuring the actual PPG signal which will be used for determining the force value of the increasing force at which the amplitude of the oscillating component of this PPG signal starts to diminish, a PPG signal can be measured over a time period of two full cycles or more, i.e., for instance, over 10 seconds or more, in order to provide enough information for assessing how the oscillating component of the PPG signal behaves due to breathing, wherein this information can be used to model the influence of the applied force on top of that.

For instance, the breathing rate can be determined by using the high-pass filtered PPG signal, i.e. the oscillating component, or the non-filtered PPG signal before applying the increasing force and/or when the application of the increasing force just started. For determining the breathing rate it is possible to, for instance, use the technique disclosed in the above mentioned article by John Allison, which is based on detecting a DC variation, an AC variation and an R-R variation, or to use other known techniques. For the modeling of the respiratory behavior of the high-pass filtered PPG signal the determined breathing rate can be extrapolated or it can be assumed that the R-R variation continues.

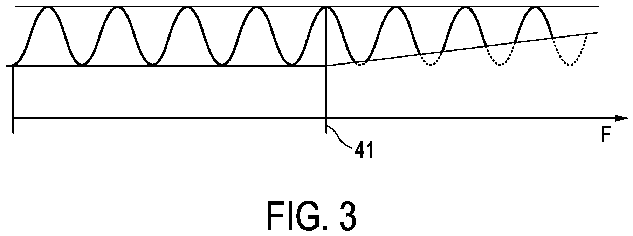

In order to determine the diastolic blood pressure of the user 30, the user puts on the wrist worn device 1 on either wrist. Preferentially, in order to output a blood pressure reading, the user sits comfortably and keeps the device 1 at heart level as illustrated in Fig. 2. The device 1 may comprise a blood pressure reading button, wherein, if the blood pressure reading button is pressed, the measurement procedure starts. During the measurement procedure the built-in force applicator pushes the PPG sensor 4 onto the skin by inflating the distensible airbag 11 enclosed in the cavity 12. The applied force stimulus preferentially linearly increases from 0 N to a predefined maximum value. Simultaneously with pushing the PPG sensor 4 onto the skin, the PPG sensor 4 registers the blood volume pulse in the skin arterioles, i.e. the PPG sensor 4 measures a PPG signal. The output of the PPG sensor 4 and the registered force applied onto the PPG sensor 4 and hence onto the skin as measured by the force sensor 13 are provided to the force value determination unit 6 which, as described above, determines a force value of the increasing force at which the amplitude of the oscillating component of the PPG signal starts to diminish in reaction to the increasing force. This is schematically and exemplarily illustrated in Fig. 3.

In Fig. 3 it can be seen that the amplitude of the oscillating component of the PPG signal 40 is firstly constant, although the applied force F is increased, wherein at a force value indicated by the line 41 the amplitude of the oscillating component of the PPG signal 40 starts to diminish. At this force value 41 the external pressure applied on the arterioles exceeds the diastolic pressure.

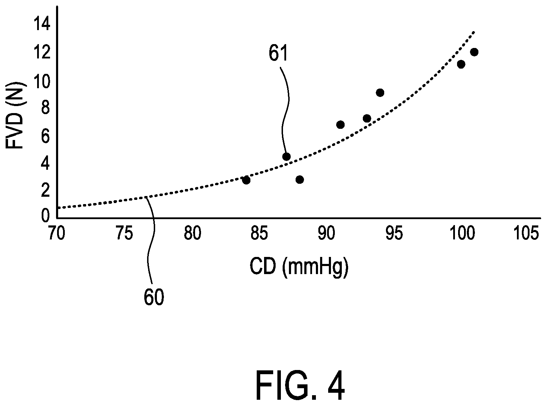

The device 1 further comprises an assignments providing unit 7 for providing assignments between force values, at which an amplitude of an oscillating component of a PPG signal starts to diminish in reaction to an increasing force, and diastolic arterial blood pressures. The assignments can be generated in a training or calibration procedure, wherein the device 1 is used for determining force values of the increasing force for different subjects, at which the amplitude of the oscillating component of the PPG signal starts to diminish in reaction to the increasing force, wherein the diastolic arterial blood pressure is known from measurements with a blood pressure cuff. Such measurements are illustrated in Fig. 4 showing the force values FVD of the increasing force at which the amplitude of the oscillating component of the PPG signal starts to diminish in reaction to the increasing force versus the diastolic arterial blood pressure CD which has been measured by using a blood pressure cuff. In Fig. 4 the points 61 are measurement point and the line 60 is an exponential fit through the points. In this example the assignments between force values FVD and diastolic arterial blood pressures CD are provided by the line 60.

The device 1 further comprises a blood pressure determination unit 8 for determining the diastolic arterial blood pressure based on the force value determined by the force value determination unit 6 and the assignments 60 provided by the assignments providing unit 7. The determined diastolic arterial blood pressure can be shown on a display 5. The display 5, the oscillating component determination unit 19, the force value determination unit 6, the assignments providing unit 7, the blood pressure determination unit 8, the breathing indicator providing unit 15 and a controller 9 for controlling the different components of the device 1 are integrated into a casing 3 arranged on the wrist band 2. After the PPG signal has been measured while the applied force has been increased, the airbag 11 is deflated.

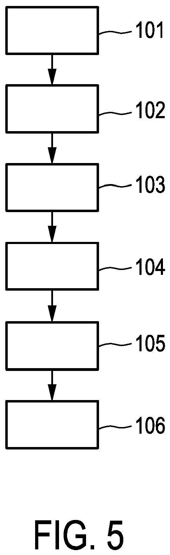

In the following an embodiment of a method for determining the diastolic arterial blood pressure of a subject will exemplarily be described with reference to a flowchart shown in Fig. 5.

In step 101 a PPG signal of the skin of the subject is measured at a measurement location by using the PPG sensor 4, while the force applicator applies an increasing force at a force application location, wherein the measurement location and the force application location are the same. In another embodiment the force application location can be proximal relative to the measurement location. Moreover, also in step 101 the force sensor 13 measures the actual increasing force applied to the skin.

In step 102 the oscillating component determination unit 19 determines an oscillating component of the PPG signal by high-pass filtering the PPG signal, and in step 103 a force value of the increasing force at which the amplitude of the oscillating component of the PPG signal starts to diminish in reaction to the increasing force is determined by using the force value determination unit. In step 104 assignments 60 between force values, at which an amplitude of an oscillating component of a PPG signal starts to diminish in reaction to the increasing force, and diastolic arterial blood pressures are provided by using the assignments providing unit 7. In step 105 the diastolic arterial blood pressure is determined based on the determined force value and the provided assignments by using the blood pressure determination unit 8. In step 106 the determined diastolic arterial blood pressure is shown on the display 5.

The device 1 and the method allow for a determination of diastolic arterial blood pressure from locally induced pressure on the skin. The PPG sensor 4 is preferentially a single wavelength reflective PPG sensor. However, in another embodiment the PPG sensor can also be a multispectral PPG sensor. Moreover, it can be a transmissive PPG sensor. The blood flow $Q$, when passing through a vessel that is too small to allow all blood to flow freely, is directly related to blood pressure $P$ through the resistance of the blood vessel $R$, expressed in the following physical relationship:

$$Q = \frac{\Delta P}{R} \, . \tag{1}$$

This means that at a certain location within a blood vessel, the blood flow through is determined by the pressure difference before and after the location. However, the resistance in the location serves as a mediator: when $\Delta P$ is kept constant but $R$ is increased, $Q$ will decrease proportional to $R$. The resistance of the vessel can be expressed in terms of the vessel properties as follows:

$$R = \frac{8L\eta}{\pi r^4} \quad , \tag{2}$$

wherein $L$ is the vessel length, $\eta$ is the blood viscosity and $r$ is the vessel radius. Thus:

$$Q = \frac{\Delta P \pi r^4}{8L\eta} \quad . \tag{3}$$

Assuming blood viscosity to be constant, the vessel radius has a polynomial relationship with blood flow.

When $r$ is decreased with a known amount and the resulting decrease in $Q$ is measured, $\Delta P$ can be computed after applying a multiplier $c$ that takes into account the remaining factors:

$$Q = \Delta P r^4 \cdot \frac{\pi}{8L\eta} = c\Delta P r^4 \quad . \tag{4}$$

It is known that $\Delta P$, the differential between pressure before and after the vessel, is not a constant but continuously modulated by the pulsating heart, which propagates a blood pulse with high pressure (i.e. systolic pressure) through the arterial system. However, even without the direct blood pulse (i.e. during diastole) there is a continuous pressure referred to as diastolic pressure that keeps blood circulating.

The PPG measurement is determined by the sum of absorptions from venous blood at very low pressure and arterial blood which increases at systole and decreases during diastole. Next to that the signal is also affected by non-blood tissue which should not be affected by pressure changes. Upon applying pressure on the skin, the following will happen. Firstly, venous blood volume will start to decrease which is expressed in the PPG signal as an increase in the baseline of the signal (overall higher reflectivity). After that, the arterial resistance will be high enough to affect the lowest level of diastolic pressure. At this point the pulsatile component of the PPG will start to shrink. This property is preferentially used by the device 1 for determining the diastolic arterial blood pressure. As the pressure on the skin is further increased, eventually the vessel resistance will be high enough to stop systolic pressure. At this point the pulse will be completely diminished and the PPG will become a non-pulsatile signal. This property is preferentially used by the device 1 for determining the systolic arterial blood pressure as will be explained in the following.

Preferentially, the force value determination unit is also adapted to determine a force value of the increasing force at which the amplitude of the oscillating component of the PPG signal becomes zero, in order to determine the systolic pressure. Correspondingly, the assignments providing unit is preferentially also adapted to provide assignments between force values, at which the amplitude of the oscillating component of the PPG signal becomes zero, and systolic arterial blood pressures. Moreover, the blood pressure determination unit is preferentially adapted to determine the systolic arterial blood pressure based on the determined force value, at which the amplitude of the oscillating component of the PPG signal becomes zero, and the provided assignments between force values, at which the amplitude of the oscillating component of the PPG signal becomes zero, and systolic arterial blood pressures. Also the systolic arterial blood pressure can be shown on the display.

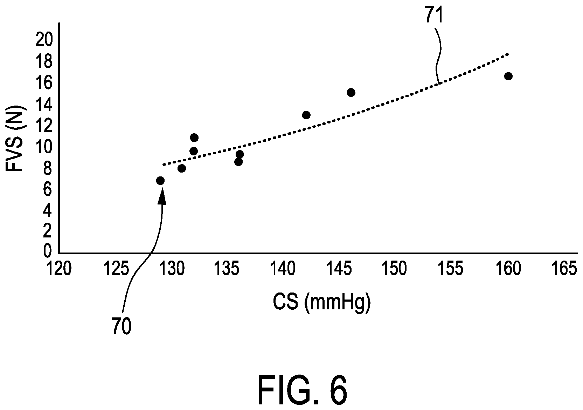

Also the assignments between the force values FVS, at which the amplitude of the oscillating component of the PPG signal becomes zero, and the systolic arterial blood pressures CS can be determined in a calibration or training procedure, wherein these force values FVS are determined while the systolic arterial blood pressures CS are known from, for instance, blood cuff measurements. Fig. 6 schematically and exemplarily illustrates such measurements 70 and a fitted line 71, wherein the assignments providing unit can be adapted to provide the fitted line 71 as the assignments to be used for determining the systolic arterial blood pressure.

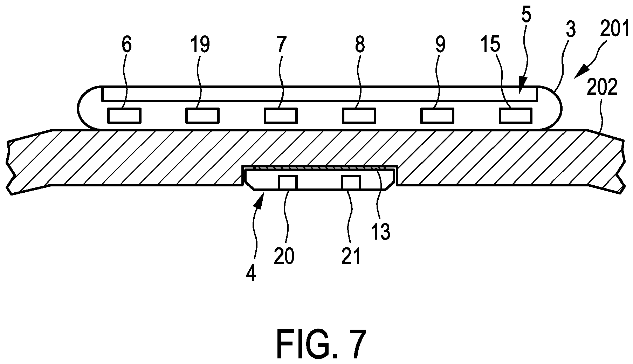

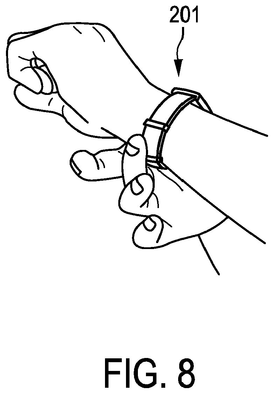

Fig. 7 shows schematically and exemplarily another embodiment of a device for determining diastolic blood pressure of a subject. The device 210 of this embodiment is similar to the device 1 described above with reference to Fig. 1, except for the force applicator, i.e. in this embodiment the increasing force is manually applied by the user by pressing on the display 5 of the device 201 as schematically and exemplarily illustrated in Fig. 8. Thus, in use the user may keep the device 201 at heart level as illustrated in Fig. 2 and then the user may apply an increasing force on the casing 3 with the display 5, while the PPG sensor 4 measures the PPG signal. While measuring the PPG signal the actually applied force is measured by using the force sensor 13, wherein this force measurement is used together with the measured PPG signal for determining the force value of the increasing force, at which the amplitude of the oscillating component of the PPG signal starts to diminish in reaction to the increasing force and wherein this force value is used by the blood pressure determination unit 8 together with the assignments provided by the assignments providing units 7 for determining the diastolic arterial blood pressure which is finally shown on the display 5. Also in this embodiment the device 201 can in addition be adapted to determine the systolic arterial blood pressure as described above based on a determination of a force value at which the amplitude of the oscillating component of the PPG signal becomes zero.

The controller 9 of the device 201 can be adapted to guide the user when exerting pressure on the casing 3 with the display 5. For instance, the controller 9 can comprise a force protocol defining a sequence of increasing force values to be applied by the user on the casing 3 with the display 5. The controller 9 can be adapted to indicate to the user that the pressure should be increased or decreased based on a) the force protocol and b) the actual force and hence the actual pressure as measured by the force transducer 13. The indication to increase or decrease the pressure can be provided via the display 5 or via another output unit. For instance, it might be provided acoustically.

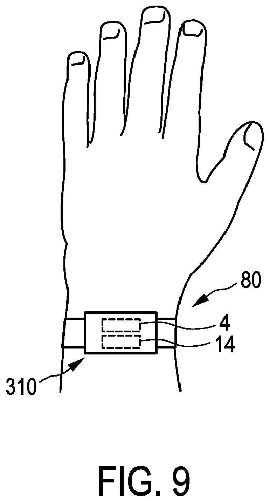

In a further embodiment of the device for determining diastolic blood pressure of a user a further PPG sensor might be provided as schematically and exemplarily illustrated in Fig. 9.

The device 310 shown in Fig. 9 can be similar to the devices 1 and 201 shown in Figs. 1 and 7, except for an additional, second PPG sensor 14 besides the PPG sensor 4 which in the following is regarded as being a first PPG sensor. The second PPG sensor 14 measures a further, second PPG signal of the skin of the user at a further measurement location, while the force applied to the skin at the force application location is increased, wherein the further measurement location is proximal relative to the force application location. In this embodiment the oscillating component determination unit 19 is adapted to determine the oscillating component of the second PPG signal by high-pass filtering and the force value determination unit 6 is adapted to determine the force value of the increasing force at which the amplitude of the oscillating component of the PPG signal starts to diminish in reaction to the increasing force by determining when a relation between the amplitude of the oscillating component of the PPG signal measured by the first PPG sensor, i.e. of the first PPG signal, and the amplitude of the oscillating component of the second PPG signal measured by the second PPG sensor starts to change. Thus, on the first PPG sensor 4 a force is applied that increases, preferentially linearly increases, from 0 N to a predefined maximum. No force is exerted on the second PPG sensor 14. The core function of the second PPG sensor 14 is to assess the influence of breathing on the oscillating component of the PPG signal measured by the first PPG sensor 4. Differences between the amplitudes of the oscillating components of the PPG signals measured by using the first PPG sensor 4 and the second PPG sensor 14 during corresponding cardiac cycles are continuously monitored. In case no force is applied, it can be assumed that fluctuations of the amplitude of the oscillating component of the first PPG signal measured by using the first PPG sensor 4 are proportional to fluctuations of the amplitude of the oscillating component of the second PPG signal measured by using the second PPG sensor 14. If the relation between the amplitude of the oscillating component of the first PPG signal and the amplitude of the oscillating component of the second PPG signal is constant within a series of consecutive cardiac cycles, it is started to apply the force on the first PPG sensor 4. At the beginning the applied force yields an external pressure on the probed arteriole wall which is below diastolic pressure in the arterioles. If the applied pressure is further increased, the ratio between the amplitudes of the oscillating components of the PPG signals measured by the first and second PPG sensors 4, 14 starts to deviate, wherein at this force value, at which this deviation starts, the applied force yields a pressure on the probed arteriole wall that exceeds the internal diastolic pressure. This force value that initiates a reduction in the amplitude of the oscillating component of the first PPG signal measured by using the first PPG sensor 4, as the external pressure applied on the probed arteriole wall exceeds the diastolic pressure, is translated to a pressure value representing diastolic arterial blood pressure by using the assignments provided by the assignments providing unit 7. The mapping from applied force to diastolic arterial blood pressure is learnt during an offline training or calibration stage as explained above.

Although in above described embodiments the PPG sensor uses green light for measuring the PPG signal, in other embodiments the PPG sensor can also be adapted to use another wavelength like red or infrared light.

Although in above described embodiments the different components of the device for determining diastolic blood pressure of a user are distributed in a certain way in the wrist band and in the casing attached to the wrist band, these components can also be distributed in another way among these components. Moreover, it is also possible that a casing or a wrist band is not present, wherein in the latter case the casing is held in place on the wrist by another attaching means. It is also possible that only a wrist band is present, wherein all components are integrated in the wrist band. Furthermore, the device can also comprise separate units which communicate with each other via a wired or wireless data connection. For instance, the components needed for carrying out the measurements can be included in a first unit being adapted to be attached to the user for carrying out the measurements, especially the measurement of the PPG signal, and the measured data can be provided to a further unit of the device, which is separate from the unit attached to the subject, wherein this further separate unit includes the components for data processing and finally determining the diastolic arterial blood pressure. For instance, this further separate unit of the device can comprise the oscillating component determination unit, the force value determination unit, the assignments providing unit and/or the blood pressure determination unit. The further separate unit can be a correspondingly adapted smartphone or another correspondingly adapted computer, particularly another handheld computer.

Although in above described embodiments the device is adapted to measure the PPG signal at a wrist, the device can also be adapted to measure the PPG signal at another part of a subject like a finger.

The oscillating component can be adapted to further process, in addition to the high-pass filtering, the PPG signal for determining the oscillating component.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like determining an oscillating component of a PPG signal, determining a force value, providing assignments, determining a diastolic arterial blood pressure, determining a systolic arterial blood pressure, et cetera performed by one or several units or devices can be performed by any other number of units or devices. For instance, the determination of the oscillating component of the PPG signal, the determination of the force value, the provision of the assignments and the determination of the diastolic arterial blood pressure can be performed by a single unit or by any other number of different units. These procedures and/or the control of the device for determining diastolic blood pressure of a subject depending on the method for determining diastolic blood pressure of a subject can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a device for determining diastolic blood pressure in an unobtrusive and painless way such that it can be determined, for instance, during sleep. A PPG signal of the skin of a subject is provided by, for instance, a PPG sensor, which has been measured at a measurement location, while a force applied to the skin at a force application location has been increased, wherein the measurement location and the force application location are the same or the force application location is proximal relative to the measurement location. A force value of the increasing force is determined by a force value determination unit, at which an amplitude of an oscillating component of the PPG signal starts to diminish in reaction to the increasing force, wherein this force value is used by a blood pressure determination unit for determining the diastolic arterial blood pressure.

**Claims**

1.  A device for determining diastolic blood pressure of a subject, the device (1) comprising:

    - a photoplethysmography (PPG) signal providing unit (4) for providing a PPG signal of the skin of the subject, which has been measured at a measurement location, while a force applied to the skin at a force application location has been increased, wherein the measurement location and the force application location are the same or the force application location is proximal relative to the measurement location,
    - an oscillating component determination unit (19) for determining an oscillating component of the PPG signal by high-pass filtering the PPG signal,
    - a force value determination unit (6) for determining a force value of the increasing force at which the amplitude of the oscillating component of the PPG signal starts to diminish in reaction to the increasing force,
    - an assignments providing unit (7) for providing assignments between force values, at which an amplitude of an oscillating component of a PPG signal starts to diminish in reaction to an increasing force, and diastolic arterial blood pressures, and
    - a blood pressure determination unit (8) for determining the diastolic arterial blood pressure based on the determined force value and the provided assignments.

2. The device as defined in claim 1, wherein the device (1) further comprises a force applicator (10, 11) for applying the increasing force to the skin while measuring the PPG signal of the skin.

3. The device as defined in claim 1, wherein the device (1) further comprises a force sensor (13) for determining the increasing force applied to the skin.

4. The device as defined in claim 1, wherein the force value determination unit (6) is adapted to only consider changes of the amplitude of the oscillating component, which are larger than a predefined threshold, for determining the force value at which the amplitude of the oscillating component of the PPG signal starts to diminish in reaction to the increasing force.

5. The device as defined in claim 1, wherein the force value determination unit (6) is adapted to determine the force value such that it is the force value of the increasing force at which the amplitude of the oscillating component of the PPG signal starts to monotonically diminish in reaction to the increasing force.

6. The device as defined in claim 1, wherein the oscillating component determination unit (19) is adapted to high-pass filter the PPG signal such that the oscillating component is the pulsatile component of the PPG signal.

7. The device as defined in claim 1, wherein the PPG signal providing unit is adapted to provide a further PPG signal of the skin of the subject, which has been measured at a further measurement location, while the force applied to the skin at the force application location is increased, wherein the further measurement location is proximal relative to the force application location, wherein the oscillating component determination unit is adapted to high-pass filter the further PPG signal for determining an oscillating component of the further PPG signal, wherein the force value determination unit (6) is adapted to determine the force value of the increasing force at which the amplitude of the oscillating component of the PPG signal starts to diminish in reaction to the increasing force by determining when a relation between the oscillating component of the PPG signal and the oscillating component of the further PPG signal starts to change.

8. The device as defined in claim 1, wherein the force value determination unit (6) is adapted to correct the amplitude of the oscillating component of the PPG signal for breathing influences and to determine the force value at which the corrected amplitude of the oscillating component of the PPG signal starts to diminish in reaction to the increasing.

9. The device as defined in claim 8, wherein the device (1) further comprises a breathing indicator providing unit (15) for providing a breathing indicator being indicative of the breathing behavior of the subject, wherein the force value determination unit (6) is adapted to determine the breathing influences based on the provided breathing indicator and to use these determined breathing influences for correcting the amplitude of the oscillating component of the PPG signal which has been measured while the force applied to the skin was increased.

10. The device as defined in claim 9, wherein the PPG signal providing unit is adapted to provide a PPG signal which has been measured before the increasing force is applied to the skin, wherein the breathing indictor providing unit (15) is adapted to determine the breathing indicator based on the provided PPG signal.

11. The device as defined in claim 9, wherein the breathing indicator providing unit comprises a breathing sensor (15) for measuring a breathing indicator.

12. The device as defined in claim 8, wherein the force value determination unit (6) is adapted to model the breathing influences based on the provided breathing indicator and to use the modeled breathing influences for correcting the amplitude of the oscillating component of the PPG signal which has been measured while the force applied to the skin was increased.

13. The device as defined in claim 1, wherein the PPG signal providing unit comprises a PPG sensor (4) being adapted to use green light for measuring the PPG signal.

14. A method for determining the diastolic arterial blood pressure of a subject, the method comprising:

- providing a PPG signal of the skin of the subject, which has been measured at a measurement location by using a PPG signal providing unit (4), while a force applied to the skin at a force application location is increased, wherein the measurement location and the force application location are the same or the force application location is proximal relative to the measurement location,
- determining an oscillating component of the PPG signal by high-pass filtering the PPG signal by using an oscillating component determination unit (19),
- determining a force value of the increasing force at which the amplitude of the oscillating component of the PPG signal starts to diminish in reaction to the increasing force by using a force value determination unit (6),
- providing assignments between force values, at which an amplitude of an oscillating component of a PPG signal starts to diminish in reaction to an increasing force, and diastolic arterial blood pressures by using an assignments providing unit (7), and
- determining the diastolic arterial blood pressure based on the determined force value and the provided assignments by using a blood pressure determination unit (8).

15. A computer program for determining the diastolic arterial blood pressure of a subject, the computer program comprising program code means for causing a device for determining the diastolic arterial blood pressure of the subject as defined in claim 1 to carry out the steps of the method for determining the diastolic arterial blood pressure of the subject as defined in claim 14, when the computer program is run on a computer controlling the device.

FIG. 1

FIG. 2

**FIG. 3**

FIG. 4

FIG. 5

FIG. 6

FIG. 7

FIG. 8

FIG. 9

Europäisches
Patentamt

European
Patent Office

Office européen
des brevets

# EUROPEAN SEARCH REPORT

Application Number

EP 17 19 1615

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/152098 A1 (UNIV MICHIGAN STATE [US]; UNIV OF MARYLAND [US]) 8 September 2017 (2017-09-08) | 1-8, 13-15 | INV. A61B5/022 A61B5/00 |
| Y | * paragraph [0024] * | 9-12 | A61B5/021 |
| | * paragraph [0058] - paragraph [0061] * | | A61B5/08 |
| | * paragraph [0075] - paragraph [0081] * | | A61B5/113 |
| | * paragraph [0100] - paragraph [0104] * | | |
| | * paragraph [0110] - paragraph [0111] * | | |
| | * paragraph [0126] * | | |
| | ----- | | |
| X | US 2017/251935 A1 (YUEN SHELTEN GEE JAO [US]) 7 September 2017 (2017-09-07) | 1-7, 13-15 | |
| | * paragraph [0002] * | | |
| | * paragraph [0054] - paragraph [0060] * | | |
| | * paragraph [0071] * | | |
| | * paragraph [0085] - paragraph [0092] * | | |
| | * paragraph [0098] * | | |
| | ----- | | |
| X | US 2005/228296 A1 (BANET MATTHEW J [US]) 13 October 2005 (2005-10-13) | 1-7, 13-15 | TECHNICAL FIELDS SEARCHED (IPC) |
| | * paragraph [0027] - paragraph [0033] * | | A61B |
| | * paragraph [0044] * | | |
| | * paragraph [0049] - paragraph [0052] * | | |
| | * paragraph [0057] * | | |
| | * figure 4A * | | |
| | ----- | | |
| Y | US 2003/036685 A1 (GOODMAN JESSE B [CA]) 20 February 2003 (2003-02-20) | 9-12 | |
| | * paragraph [0152] - paragraph [0156] * | | |
| | * paragraph [0177] - paragraph [0179] * | | |
| | * paragraph [0237] - paragraph [0238] * | | |
| | ----- | | |
| | -/-- | | |

The present search report has been drawn up for all claims

| Place of search | Date of completion of the search | Examiner |
|---|---|---|
| The Hague | 20 February 2018 | Gooding Arango, J |

EPO FORM 1503 03.82 (P04C01)

page 1 of 2

Europäisches
Patentamt

European
Patent Office

Office européen
des brevets

**EUROPEAN SEARCH REPORT**

Application Number

EP 17 19 1615

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SARAH SCHNEIDER ET AL: "Blood pressure measurement on the cheek", CURRENT DIRECTIONS IN BIOMEDICAL ENGINEERING, vol. 2, no. 1, 30 September 2016 (2016-09-30), XP055452456, DOI: 10.1515/cdbme-2016-0053 * the whole document * ----- | 1-15 | |

TECHNICAL FIELDS
SEARCHED      (IPC)

The present search report has been drawn up for all claims

| Place of search | Date of completion of the search | Examiner |
|---|---|---|
| The Hague | 20 February 2018 | Gooding Arango, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

## ANNEX TO THE EUROPEAN SEARCH REPORT
## ON EUROPEAN PATENT APPLICATION NO.

EP 17 19 1615

This annex lists the patent family members relating to the patent documents cited in the above-mentioned European search report.
The members are as contained in the European Patent Office EDP file on
The European Patent Office is in no way liable for these particulars which are merely given for the purpose of information.

20-02-2018

| Patent document cited in search report | Publication date | Patent family member(s) | Publication date |
|---|---|---|---|
| WO 2017152098 A1 | 08-09-2017 | NONE | |
| US 2017251935 A1 | 07-09-2017 | NONE | |
| US 2005228296 A1 | 13-10-2005 | US 2005228296 A1<br>US 2005228299 A1<br>US 2005245831 A1 | 13-10-2005<br>13-10-2005<br>03-11-2005 |
| US 2003036685 A1 | 20-02-2003 | US 6616613 B1<br>US 2003036685 A1 | 09-09-2003<br>20-02-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Photoplethysmography and its application in clinical physiological measurement. **JOHN ALLEN.** Physiological Measurement. IOP Publishing, 2007, vol. 28, R1-R39

- Developing an algorithm for pulse oximetry derived respiratory rate (RRoxi): a healthy volunteer study. **PAUL S. ADDISON et al.** Journal of Clinical Monitoring and Computing. 2012, vol. 26, 45-51